(19) Europäisches Patentamt
European Patent Office
Office européen des brevets

(11) EP 3 246 705 A1

(12) **EUROPEAN PATENT APPLICATION**

(43) Date of publication:
**22.11.2017 Bulletin 2017/47**

(51) Int Cl.:
***G01N 33/557*** *(2006.01)*

(21) Application number: **17171447.0**

(22) Date of filing: **17.05.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **18.05.2016 US 201615158374**

(71) Applicant: **Sapidyne Instruments Inc.**
**Boise, ID 83705 (US)**

(72) Inventors:
- **LACKIE, Steve J.**
  **BOISE, ID 83705 (US)**
- **GLASS, Thomas R.**
  **BOISE, ID 83705 (US)**

(74) Representative: **Boggio Merlo, Anita et al**
**Dragotti & Associati S.r.l.**
**Via Nino Bixio, 7**
**201292 Milano (IT)**

(54) **IMPROVED BINDING ASSAY**

(57) What has been developed is an improved method of determining the binding constants between two molecules that requires significantly fewer materials and potentially less time (in the case of a whole cell analysis less time to grow cell cultures as fewer cells are required in the same analysis) to undertake. The method involves utilizing an NSB measurement in preferably an n-curve analysis in order to determine the $K_d$ and/or $R_t$ without having to complete actual measurements to determine the lower knee of the curve(s) in the n-curve analysis. Preparing the experiment utilizing the additional samples allows an experiment represented in a binding curve having an upper and a lower knee to no longer be required to run through the lower knee to a point of completion.

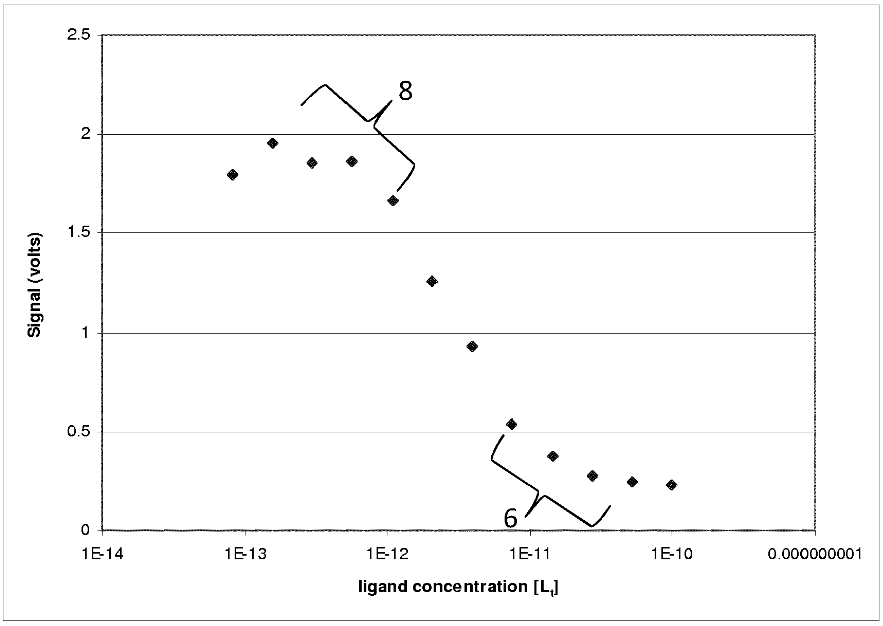


FIG. 1.2

EP 3 246 705 A1

## Description

In the context of analyzing receptor-ligand interaction, binding is the joining of two entities (molecules) that have some affinity for each other. This binding is almost always reversible, meaning the two molecules (generically known as ligand and receptor) will join together and come apart over and over again.

In general, these molecules associate (or bind), remain linked together for a while, and then dissociate (come apart). Eventually, an equilibrium state is reached such that the concentration of bound partners is no longer changing. Association and dissociation continue, but since the concentrations are no longer changing, the number of association events per second must equal the number of dissociation events per second.

Receptor and ligand association is important in a variety of biochemical and medical fields. Receptor and ligand association/disassociation rates play a very important role, for example, in the development of pharmaceutical drugs. The rates at which a drug interacts with its target protein in-vivo affects the duration for which the drug is active and the strength of the drug activity, and how the drug interacts with the organism's physiological system.

The disassociation constant ($K_d$) is a common way to describe the affinity between two molecules, which for the purpose of this document are called a receptor (R) and a ligand (L). $K_d$ is defined as follows in Equation 1:

$$K_d = \frac{[R]\cdot[L]}{[RL]}$$

Equation 1.

Expressions representing conservation of mass are given below in Equation 2:

$$[R_{total}] = [R] + [RL]$$

$$[L_{total}] = [L] + [RL]$$

Equation 2.

When Equations 1 and 2 are combined, the resulting equation provides for an equation to determine the expression for the free receptor [R] in an interaction. Alternatively the amount of bound receptor can be measured and represented as [RL] (=[$R_{total}$]-[R]) in the equation by substituting bound receptor for free receptor in the above and following equations:

$$[R] = \frac{-1}{2}\cdot K_d - \frac{1}{2}\cdot\left[L_t\right] + \frac{1}{2}\cdot\left[R_t\right] + \frac{1}{2}\cdot\left[K_d^{\,2} + 2\cdot K_d\cdot\left[L_t\right] + 2\cdot K_d\cdot\left[R_t\right] + \left(\left[L_t\right]\right)^2 - 2\cdot\left[L_t\right]\cdot\left[R_t\right] + \left(\left[R_t\right]\right)^2\right]^{\frac{1}{2}}$$

Equation 3.

Depending on experimental convenience, either the amount of free or bound receptor in a solution can be determined by measuring a signal (typically the voltage representing the flourescence of a tag or label applied to a molecule) that is proportional to the amount of free (or bound) receptor in the solution. This concept is expressed in the following equations in which Sig100 is the signal when the free receptor is equal to the total amount of receptor ([R]=[$R_t$]) and the non specific binding (NSB) is the signal when the receptor is fully saturated with ligand ([RL]=[$R_t$]):

$$\text{Signal} = \frac{(\text{Sig100} - \text{NSB})}{[R_t]} \cdot [R] + \text{NSB}$$

Equation 4.

Substituting [R] from Equation 3 into Equation 4 provides the following Equation 5:

$$\text{Signal} = \frac{(\text{Sig100} - \text{NSB})}{[R_t]} \cdot \left[ \frac{-1}{2} \cdot \left[ K_d + [L_t] - [R_t] - \left[ K_d^{\,2} + 2 \cdot K_d \cdot [L_t] + 2 \cdot K_d \cdot [R_t] + ([L_t])^2 - 2 \cdot [L_t] \cdot [R_t] + ([R_t])^2 \right]^{\frac{1}{2}} \right] \right] + \text{NSB}$$

Equation 5.

Using Equation 5 in conjunction with a series of signals measured with a variety of $L_t$ concentrations and a single $R_t$ concentration, an analysis is performed in which the values of Sig100, NSB, $K_d$ and $[R_t]$ are varied iteratively to determine the best fit of the variables of the equation to the measured signals.

In Figure 1.1, a sample set of data is provided in which known total ligand concentrations in the left column have been added to a series of identical receptor solutions. After the receptor and ligand have been allowed to come to a binding equilibrium, a signal measurement is taken that represents the free receptor concentration. The data from Figure 1.1. is provided in a graph (Figure 1.2) depicting the signal in volts on the Y axis representing the signal in equations 4 and 5 above plotted against the total ligand concentration $[L_t]$ that was added to the receptor. As illustrated in Figure 1.2, the data points present a general curve from which two "knees" 6, 8 are apparent.

Analysis of the data of Figures 1.1 and 1.2 can proceed in several alternative ways. First, the values of $L_t$ can be treated as known values and values of Sig100, NSB, $R_t$, and $K_d$ are determined to give a best fit of equation 5 to the data. Second, the values of $R_t$ can be treated as known values and the values of Sig100, NSB, $L_t$ (varied together in a fixed relationship to each other) and $K_d$ are determined to give a best fit of equation 5 to the data as depicted by curve 18. Third, both $R_t$ and $L_t$ can be treated as known values and Sig100, NSB, and $K_d$ are varied to find a best fit to the data.

Error graphs may be used to evaluate the uniqueness of fit of a parameter found as a best fit value in the procedure outlined. Error graphs are plots of the residual error (which may be expressed as a percentage of the specific binding signal) computed for a range of alternative values of a given parameter as illustrated in Figures 4.1-5.3. For example, the graph in Figure 6 depicts an error curve illustrating a potential range in which the $K_d$ is located within a certain confidence level. Note that the minimum in Figure 6 occurs at the best fit value of $K_d$ determined as described above and that the other three parameters (Sig100, NSB, and $R_t$ in this case) have been reoptimized for each alternate value of $K_d$ plotted.

Notice that the error graphs computed for the data of Figure 1.1 and 1.2, depicted in Figure 7, show that only the $K_d$ can be determined with any accuracy, whereas the $R_t$ result only provides that the $R_t$ is below a certain number (hence the flat portion of the line on the left side of the graph). This result is typical for a curve measured with an Rt value near to or below the $K_d$ value. Conversely, in an experiment conducted at a $R_t$ concentration far greater than the $K_d$ as depicted in Figure 8, the $R_t$ can be resolved within a reasonable certainty but the $K_d$ is only depicted as being less than a calculated number (less than 298pM in Figure 8).

In order to determine both the $K_d$ and the $R_t$, an n-curve analysis of two or more curves is typically utilized as shown in Figure 9. In a two curve analysis the user alters the concentration of the receptor in the second set of measurements (the receptor is fixed across each set of measurements but varied between the sets of measurements) to provide for two curves. Utilizing two curves in the n-curve analysis provides for resolution of both the $R_t$ 34 and the $K_d$ 36.

As discussed above several options exist in the choice of parameters to vary and parameters to hold fixed. Generally, a researcher chooses to hold fixed the parameter value that is most accurately known.

The above methodology can be applied to measuring the interaction between two molecules in which one of the molecules is located on the surface of a cell. These "whole cell measurements" are important in that the measurements are thought to more accurately represent interactions found in vivo. To perform a whole cell $K_d$ measurement, typically a cell culture is grown in which the ligand is expressed on the cell surface (note: as described above ligand and receptor are generic terms and ligand on the cell may well be a cell surface receptor). A suspension of a known number of cells is typically made and subsequently a series of dilutions is performed to obtain a series of volumes of cell suspensions in which the concentration of cells is titrated. Each suspension in the series of known dilutions is then mixed with a single complimentary binding partner (receptor) concentration. The titrations are then incubated to allow the interaction between the ligand and the receptor to come to equilibrium. Typically the incubation occurs on a rocker to keep the cells in suspension. After equilibrium, the cells are centrifuged into a pellet. The remaining supernatants (containing the free receptor) are removed and measured for the concentration of receptor present. In analysis of such experiments it is most common to hold the receptor concentration (the soluble binding component) fixed and vary the effective concentration of the ligand (the cellularly expressed binding partner). This analysis choice enables calculation of the $R_t$ of the ligand on the cell surface.

In general a dual curve (or any number greater than a single curve, thus an n-curve) analysis is utilized in whole cell measurements. Two or more series of cell dilutions are prepared and titrated into two or more different, known receptor concentrations.

The dual (or greater) curves are typically obtained simultaneously to allow for the same cell culture to be used thus providing for the same cell $R_t$ for all curves. The spacing between the multiple curves allows for further definition of the $K_d$ and $R_t$ by providing an additional parameter that further constrains the concentration and Kd variables. While one curve can be utilized in the event the cell expression ($R_t$) level is known, an n-curve analysis is typically necessary to avoid a finding of ambiguity in either the $K_d$ or in the cell expression level. Further, if only one curve is initially used and the result leads to ambiguity in either the $K_d$ or in the cell expression level, it may not be practical to add a second curve because the cell expression level may change from culture to culture and from time to time.

A typical whole cell n-curve analysis utilizing two curves is shown in Figure 10. The dual curve graph charts the percent free of the receptor along the x-axis while the concentration illustrates the concentration of the cells (e.g. cells/ml) in the titrated sample. Figure 11 constitutes the raw data from each curve depicted in Figure 10. As shown from the data of Figure 11, the upper knee of the curves are composed of cell dilutions 64, 70 that contain significantly fewer cells per mL than at the lower knee of the curves 66, 68. This range is from 30 million cells per mL down to 1,830 cells per mL. As illustrated by the error curves regarding the $K_d$ 56 and $R_t$ 58, the experiment has resolved the measurements with fair certainty.

While the experiment in Figures 10-11 depicts accurate data that can be obtained by utilizing a significant number of cells, in practice this approach often encounters difficulties that either limit its application or render the application unusable. Limitations on the approach include, but are not limited to, the following: cells can be difficult, expensive, and/or time consuming to culture into large numbers; the concentration of cells needed for saturation of the receptor (the lower plateau) may be effectively solid, particularly if the cells exhibit a low expression level of the targeted ligand and/or a weak interaction between the ligand and receptor. These factors often limit the applicability of the experiment as researchers may not be inclined to invest the time and monetary resources into an experiment that may not have a clear resolution.

Figure 12 depicts a whole cell dual curve analysis in which the data from the second knee of the curve has not been analyzed in determining the $K_d$ and $R_t$ of the receptor on the cell surface. In the depicted experimental curve the data from the lower knee of the curves is ignored (as shown in Figures 13.1 and 13.2 as ignored data 82, 84) to simulate a researcher not obtaining experimental data through the lower knee of the curve. The failure of the curves to resolve into two distinct knees depicts what was previously considered the necessity of obtaining measurements in the full range of the curve. The shape of the error curves depict that the $K_d$ 76 and $R_t$ 78 have not been resolved with reasonable certainty due to the absence of measurement to accurately obtain the lower knees of the curve. In addition to Figure 12 and the associated data illustrated in Figures 13.1 and 13.2 depicting the procedure for and necessity of obtaining both knees of the curve(s) is further referenced in literature in the field. For example, prior publications have stated "[t]o obtain complete experimental curves that spanned the range between 0% and 100% free antibody signals, two-fold serial dilutions of cells were made ..." ("High Affinity Binding Measurements of Antibodies to Cell-Surface-Expressed Antigens, "Rathanaswami et. al, 1 Anal. Biochem., (2008) 52-60)) and "[f]or example, to measure a 1 nM affinity, the starting antigen concentration present on cells should be in the range of hundreds of nanomolar to provide saturation and obtain the lower end of the $K_d$ curve." ("Measurement of the functional affinity constant of a monoclonal antibody for cell surface receptors using kinetic exclusion flourescence immunoassay," Xie et. al, J. Immunological Methods, 304 (2005) 1-14)).

## Summary of the Invention

What has been developed is an improved method of determining the binding constants between two molecules that requires significantly fewer materials and potentially less time (in the case of a whole cell analysis less time to grow cell cultures as fewer cells are required in the same analysis) to undertake. The above described experiment no longer needs to be run through the full range of data values to establish both knees of higher and lower concentration of the curve(s). The method involves utilizing a saturated receptor measurement in preferably an n-curve analysis in order to determine the $K_d$ and/or $R_t$ without having to complete actual measurements to determine the lower knee of the curve(s) in the n-curve analysis. The method is thought to work to save time and materials (and therefore money) both in a whole cell analysis or alternatively in an n-curve analysis in which the molecules are weak binders thus requiring a high concentration of materials to determine the $K_d$ and/or to save materials in the event the materials are scarce and/or expensive.

In the above description of the theory behind the invention the terms R and L are utilized to describe Receptor and Ligand. It is important to note that in the above theory the terms R and L are interchangeable for the purpose of the analysis. Because of this interchangeability, in the following description and in the claims the terms A and B are utilized to describe the molecules in the interaction, with either A or B being utilized to describe a receptor. Similarly, the terms A or B can be utilized to describe a ligand. The method detailed below is for determining binding constants for a molecular interaction between a first binding partner A and a second binding partner B. The first binding partner A and the second binding partner B reversibly bind to form complex AB.

The method involves a series of steps, including the step of providing two or more solutions of molecule B. In a preferred embodiment this step involves providing a plurality of first solutions having molecule B and providing a plurality of second solutions having molecule B. In a preferred embodiment the plurality of first solutions of molecule B have a uniform known concentration of molecule B across the plurality of first solutions. In a preferred embodiment the plurality of second solutions of molecule B have the same known concentration of molecule B across the plurality of second solutions. In a preferred embodiment the concentration of molecule B across the plurality of first solutions differs from the concentration of molecule B across the plurality of second solutions.

The method includes the step of providing a first NSB solution that is free of molecule B and is kept free of molecule B. This measurement can either include molecule A or be free of molecule A.

The method includes the step of adding a known amount of molecule A to the first solution and a known amount of molecule A to the second solution. In an embodiment the amount of molecule A added to the first solution is different than the known amount of molecule A added to the second solution. In a preferred embodiment this step involves adding a varying known amount of molecule A to each of first solutions in the plurality of first solutions and adding a varying known amount of molecule A to each of the second solutions in the plurality of second solutions. Alternatively molecule A can be added to the first and second solutions of molecule B sequentially or serially.

After the addition of molecule A to the solutions in the plurality of first and second solutions, the solutions are incubated, which allows for molecule A to bind to molecule B. In a preferred embodiment molecule A or molecule B is a molecule expressed on the surface of a cell. Molecule A or B can be expressed on the surface of the cell either as part of the cell's natural expression, or the cell can be engineered to express or over express molecule A or B on the surface of the cell. In an alternate embodiment in which molecule A is added to the first and second solutions of molecule B sequentially or serially, the solutions are allowed to incubate after each addition of molecule A.

After incubating the first and second solutions which contain molecule A and molecule B, the signals of free or bound molecule B in the first solutions and the second solutions are measured. In a preferred embodiment the solutions are allowed to incubate until reaching equilibrium. A measurement of the signal of free or bound molecule B in the first NSB solution is also taken. In general the measurements of free or bound can be considered as interchangeable as the computation can be made with either a direct measurement of free molecule, or with a measurement of the amount of bound molecule subtracted from the total, which would provide for the amount of free molecule in the solution.

From the measurements of the signal of free or bound molecule B in the first solutions, second solutions, and first NSB solution, the binding constants for the molecular interaction between the molecule A and the molecule B are then calculated. The binding constants are calculated, as discussed above but utilizing the measurement of the first NSB solution, by comparing a function utilizing the binding constants between molecule A and molecule B, the concentration of molecule B in the first solutions and the second solutions, the concentration of molecule A added to each of the plurality of first solutions and plurality of second solutions, the signal of the solution at 100% free or bound molecule B (Sig100), and the signal of the non specific binding (NSB) from the first solution and the second solution by varying the actual and/or theoretical values of the concentration of molecule B, $Sig_{100}$, NSB, and the binding constants to the obtained results to obtain the binding constants of best fit in the calculation, but then further utilizing the signal measured for free or bound molecule B in the first NSB solution to define the signal from solutions 1 and 2 in the presence of saturating quantity of molecule A. It is further important to note that the experimental design can vary without departing from the inventive concepts disclosed herein.

The measurement signal obtained from the first NSB solution can be utilized in several different potential mechanisms in the analysis. The measurement can be used as an actual NSB measurement in analyzing the signals from the first and second solutions. In the event that a second NSB solution is used in which the second NSB solution is free of molecule B but otherwise identical to the second solution and the first NSB solution is free of molecule B but otherwise identical to the first solution, the signal of free molecule B from the first NSB and second NSB solutions can be used as the actual NSB value for the first solution and the second solution, respectively. Additional solutions in similar concept can be added to the experimental design with the option of utilizing a further NSB solution for one or more of the additional solutions added.

Alternatively, the signal measured from the first NSB solution can be utilized as a data point to compare to the theoretical NSB value to determine an NSB value of best fit in calculating the binding constants. Similarly, a second NSB solution can be provided: the signal from the first NSB solution can be utilized as a data point to compare to the theoretical NSB value to determine an NSB value of best fit for the first solution, the signal measured from the second NSB solution would be used as a data point to compare to the theoretical NSB value to determine an NSB value of best fit for the second solution.

Alternatively the signal measured for free molecule B in the first NSB solution can be used to define a lower plateau of measurement lacking free molecule B. The NSB is then calculated by assigning a significantly greater concentration of molecule A to the signal from the first NSB solution than added to the solutions in the step of adding a known amount of molecule A to the first and second solutions. The assigned concentration of molecule A is made high enough to simulate a solution in which effectively 100% of molecule B is bound to molecule A.

The purpose of the Summary is to enable the public, and especially the scientists, engineers, and practitioners in the art who are not familiar with patent or legal terms or phraseology, to determine quickly from a cursory inspection, the nature and essence of the technical disclosure of the application. The Summary is neither intended to define the invention of the application, which is measured by the claims, nor is it intended to be limiting as to the scope of the invention in any way.

**Brief Description of the Figures**

Figure 1.1 is a table of a sample of binding signal obtained from free receptor after a series of solutions of ligand receptor of uniform concentration have been combined and allowed to equilibrate with a series of solutions with varying receptor concentration.

Figure 1.2 is a graph of the information provided in table 1.1.

Figure 2.1 is a chart of theoretical signals and associated error calculated from the actual signal recorded in Figure 1.1.

Figure 2.2 is a graph of the theoretical and actual signals from Figure 2.1.

Figure 3.1 is a table illustrating further iterative analysis of the data presented in Figure 2.1.

Figure 3.2 is a table illustrating further iterative analysis of the data presented in

Figure 2.1 and resulting in a line graph presenting the lowest RMS error of the combined theoretical data.

Figure 3.3 illustrates the results of the theoretical data with the lowest RMS error.

Figure 4.1 illustrates the first step in plotting an error curve for the $K_d$ data obtained in the iterative analysis described above.

Figure 4.2 illustrates the data of the first $K_d$ value plotted in Figure 4.1.

Figure 5.1 illustrates the actual data versus theoretical signal curve derived from the data in Figure 3.1 but in which the $K_d$ value has been varied to provide a theoretical error percent.

Figure 5.2 illustrates the plotting of the second $K_d$ value on the $K_d$ error graph of Figure 4.1.

Figure 5.3 illustrates a second $K_d$ value plotting the error graph as shown in Figure 5.2.

Figure 6 illustrates a $K_d$ value error graph for determining a range in which the $K_d$ has been determined to exist and the error associated with the values in that range.

Figure 7 illustrates an example determination of a $K_d$ for a single curve analysis and the determination of the $K_d$ and $R_t$ from the data depicted in the line graph. The $K_d$ has been resolved with reasonable certainty while the $R_t$ has not been resolved with reasonable certainty as evidenced by the lack of curvature in the left side of the graph.

Figure 8 illustrates an example determination of a $K_d$ for a single curve analysis and the determination of the $K_d$ and $R_t$ from the data depicted in the line graph. The $R_t$ has been resolved with reasonable certainty while the $K_d$ has not been resolved with reasonable certainty as evidenced by the lack of curvature in the left side of the graph.

Figure 9 illustrates a dual curve analysis and the ability of the dual curve analysis to resolve both the $R_t$ and the $K_d$.

Figure 10 illustrates a dual curve whole cell analysis in which measurements have been obtained for data through both knees of the curve.

Figures 11.1-11.2 illustrate the data measurements obtained and displayed in the graphed curves of Figure 10.

Figure 12 illustrates a theoretical dual curve whole cell analysis in which the experiment has not run through the second knee in the curve. For illustration purposes the data are the data utilized in Figure 10 but with data through the second knee of the curve ignored in the analysis. The error curves illustrate that neither the $K_d$ nor the $R_t$ of the receptor on the cell surface can be determined without obtaining a full curve.

Figures 13.1-13.2 illustrate the data plotted in the dual curve analysis of Figure 12 illustrating the data from the second knee are ignored in the analysis.

Figure 14 illustrates the dual curve whole cell analysis depicted in Figures 10 and 12 but in which a theoretical third solution has been measured to provide an NSB measurement utilized according to the inventive concept of this application. The use of the data point has allowed the $K_d$ and $R_t$ to be resolved.

Figures 15.1-15.2 illustrates the dual curve analysis data used in Figure 14 but with NSB points 96, 97 utilized in the graph of Figure 14 and associated analysis.

Figure 16 further illustrates the dual curve analysis data of Figures 14 but with an additional data point utilized that allows further resolution of the $K_d$ and $R_t$.

Figures 17.1-17.2 illustrates the dual curve analysis data used in Figure 16 but with additional actual data points 99, 101 considered in the analysis.

**Detailed description of the Figures Depicting the Inventive Concept**

An example of the concept described in the above Background section is provided in Figures 1.1-9.

Figures 14-17.2 depict a whole cell dual n-curve analysis according to the above experimental description but in which the lower knee of each curve has not been determined through experimental measurement and subsequent iterative analysis as described above. Instead, an NSB measurement taken from a solution free of the receptor has been utilized in the iterative analysis described above. Utilizing the NSB measurement the analysis was able to resolve the curves 85, 86 as though actual measurements had been taken through the entire curve range and lower knee of the curve without actually having to utilize the full range of materials and time needed to conduct the experiment through these values. The analysis provided for a resolved $K_d$ 81 and $R_t$ 83 both with reasonable certainty and comparable to the $K_d$ and $R_t$ determined in Figure 12. The benefit of this analysis is it allowed far fewer cells to be utilized in the experimental analysis. As illustrated in Figures 11.1 and 11.2, the experiment to determine full curve range required the experiment to proceed through a curve point having a cell concentration of 30.0M Cells/mL 65, 67. In contrast, experiments depicted in Figures 14-17.2 depict that it is thought that the experiment only need to run through a point to obtain the upper curve and including an NSB point in the analysis allows the analysis to resolve the full curve with a reasonable certainty.

The point to which the experiment ran in Figures 14 and 16 went through 234.38k cells/mL 95, 97 or 468.75k cells/mL 99, 101. This data shows that only approximately 0.8% or 1.6%, respectively, of the total number of cells was needed to resolve the experiment by including the measurement of the first NSB solution free of molecule B (which in this case was the ligand) and still obtaining a $K_d$ value 81 within reasonable certainty and $R_t$ within reasonable certainty 83. This data represent a potential huge savings in time and resources required to grow a cell culture (far fewer cells can be used in the new analysis), potential large monetary savings from a researcher having to purchase far fewer cells, and/or large savings in non-whole cell analysis in which either or both of the binding molecules are expensive, precious, or both. The data further represent the ability to measure a system in which the concentrations needed to reach the second knee cannot be achieved because of the low expression level of a molecule on a cell surface and/or weak interaction between the molecules.

**Claims**

**1.** A method for determining binding constants for a molecular interaction between a first binding partner A and a second binding partner B, wherein said first binding partner and said second binding partner form complex AB, wherein said method comprises the following steps:

the step of providing at least a first solution comprising molecule B and at least a second solution comprising molecule B;

the step of providing a first NSB solution, wherein said first NSB solution is free of molecule B;

the step of adding a known amount of molecule A to said first solution and adding a known amount of molecule A to said second solution and incubating said first solutions and said second solution after adding said molecule A;

the step of measuring the signal of free molecule B in said first solution and said second solution after allowing said first solutions and said second solutions to incubate;

the step of measuring the signal of free molecule B in said first NSB solution; and

the step of calculating the binding constants for the molecular interaction between said molecule A and said molecule B wherein said binding constants are calculated by comparing a function comprised of the binding constants between molecule A and molecule B, the concentration of molecule B in said first solution and said second solution, the concentration of molecule A added to each of said first solution and said second solutions, the signal of the first solution and the second solution at 100% free molecule B (Sig100), and the signal of the non specific binding (NSB) from said first solution and said second solution by varying the actual and/or theoretical values of the concentration of free or bound molecule B, $Sig_{100}$, NSB, and the binding constants to the obtained results to obtain the binding constants of best fit in said calculation, wherein said step further comprises utilizing the signal measured for free or bound molecule B in said first NSB solution to define the signal from solutions 1 and 2 in the presence of saturating quantity of molecule A.

2. The method of determining binding constants for a molecular interaction between a first binding partner A and a second binding partner B of claim 1, wherein said step of providing a first solution comprising molecule B and a second solution comprising molecule B comprises providing a plurality of first solutions comprising molecule B and a plurality of second solutions comprising molecule B, wherein said plurality of first solutions of molecule B comprise the same known concentration of molecule B, wherein said plurality of second solutions comprise the same known concentrations of molecule B, wherein said concentration of molecule B is non-identical in said first solution and said second solution.

3. The method of determining binding constants for a molecular interaction between a first binding partner A and a second binding partner B of claim 2 wherein the step of adding a known amount of molecule A to said first solution and a known amount of molecule A to said second solution comprises adding varying known amounts of molecule A to each of said first solutions in said plurality of first solutions and varying known amounts of molecule A to each of said second solutions in said plurality of second solutions and incubating said plurality of first solutions and said plurality of second solutions after adding said molecule A.

4. The method of determining binding constants for a molecular interaction between a first binding partner A and a second binding partner B of claim 1, wherein said molecule A or said molecule B comprises a molecule expressed on a surface of a cell.

5. The method of determining binding constants for a molecular interaction between a first binding partner A and a second binding partner B of claim 1, wherein said step comprising incubating said first solution and said second solution comprises incubating said first solution and said second solution until the molecular interaction between molecule A and molecule B in each of said solutions reaches equilibrium.

6. The method of determining binding constants for a molecular interaction between a first binding partner A and a second binding partner B of claim 1, wherein said step of calculating the binding constants for the molecular interaction between said molecule A and said molecule B by including the signal measured for free molecule B comprises utilizing said signal of free molecule B from said first NSB solution as an actual NSB measurement in analyzing the signals from said first and second solutions.

7. The method of determining binding constants for a molecular interaction between a first binding partner A and a second binding partner B of claim 1, wherein said method comprises the step of adding a second NSB solution, wherein said second NSB solution is free of molecule B, wherein said first NSB solution and said second NSB solution are identical to said first solution and said second solution, respectively, but for said first NSB solution and said second NSB solution are free of molecule B; and
wherein said step of calculating said binding constants comprises utilizing the signal of free molecule B from said first NSB solution and said second NSB solution as the actual NSB value for the first solution and the second solution, respectively.

8. The method of determining binding constants for a molecular interaction between a first binding partner A and a second binding partner B of claim 1, wherein said step of calculating binding constants comprises utilizing said signal measured from said first NSB solution as a data point to compare to the theoretical NSB value to determine an NSB value of best fit in calculating said binding constants.

9. The method of determining binding constants for a molecular interaction between a first binding partner A and a second binding partner B of claim 8, wherein said method comprises the step of providing a second NSB solution free of molecule B, wherein said second NSB solution comprises an identical solution to said second solution but for said second NSB solution is free of molecule B, wherein said first NSB solution comprises an identical solution to said first solution but for said first NSB solution is free of molecule B, wherein said step of calculating binding constants comprises utilizing said signal measured from said first NSB solution as a data point to compare to the theoretical NSB value to determine an NSB value of best fit for said first solution, and wherein said step of calculating binding constants comprises utilizing said signal measured from said second NSB solution as a data point to compare to the theoretical NSB value to determine an NSB value of best fit for said second solution.

10. The method of determining binding constants for a molecular interaction between a first binding partner A and a second binding partner B of claim 1, wherein said step of calculating the binding constants for the molecular interaction between said molecule A and said molecule B further comprises utilizing the signal measured for free molecule B in said first NSB solution to define a lower plateau of measurement lacking free molecule B, wherein the NSB is calculated by assigning a significantly greater concentration of molecule A than was added to said solutions in said step of adding a known amount of molecule A to said first and second solutions to simulate a solution in which 100% of molecule B is bound to molecule A.

**11.** The method of determining binding constants for a molecular interaction between a first binding partner A and a second binding partner B of claim 10, wherein said step of providing a first NSB solution comprises providing at least a second NSB solution, wherein said step of calculating the binding constants further comprises utilizing the signal measured for free molecule B in said NSB solutions to define a lower plateau of measurement lacking free molecule B, wherein the NSB is calculated by assigning a significantly greater concentration of molecule A than was added to said solutions in said step of adding a known amount of molecule A to said first and second solutions to simulate a solution in which 100% of molecule B is bound to molecule A.

| Concentration | Signal |
|---|---|
| 1.00E-10 | 0.23 |
| 5.24E-11 | 0.25 |
| 2.75E-11 | 0.28 |
| 1.44E-11 | 0.38 |
| 7.56E-12 | 0.54 |
| 3.96E-12 | 0.93 |
| 2.08E-12 | 1.26 |
| 1.09E-12 | 1.66 |
| 5.72E-13 | 1.87 |
| 3.00E-13 | 1.86 |
| 1.57E-13 | 1.96 |
| 8.24E-14 | 1.80 |
| 0.00E+00 | 1.64 |

**FIG 1.1**

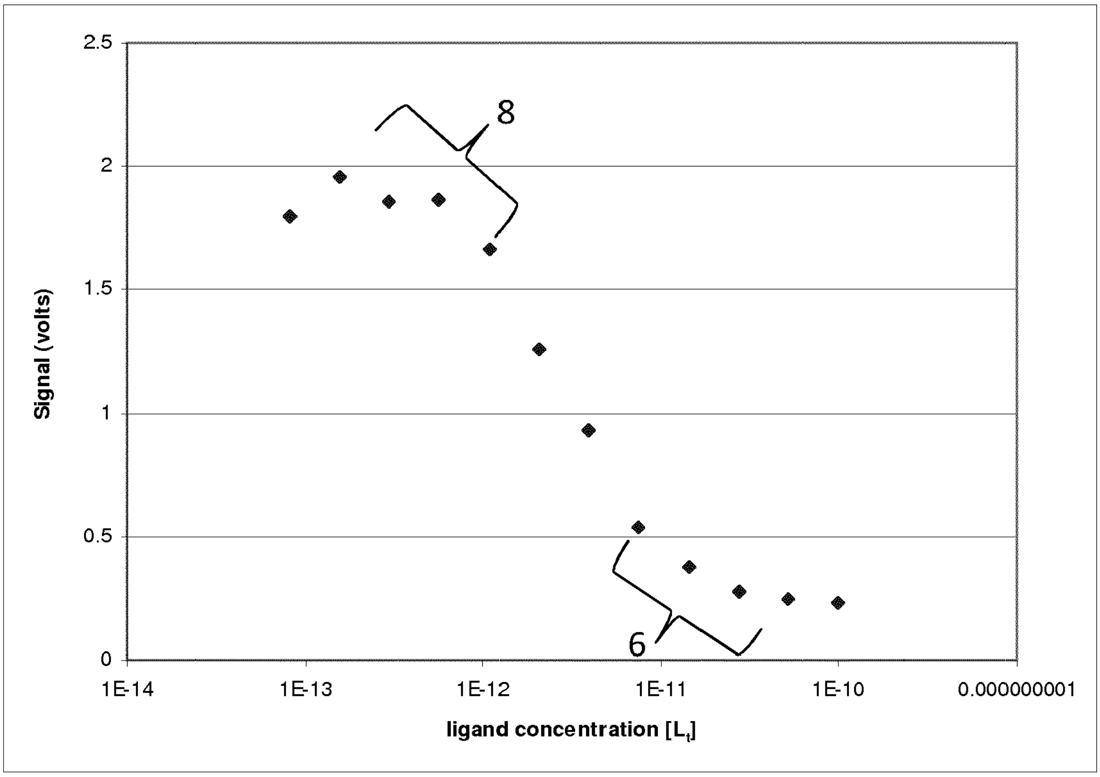


**FIG. 1.2**

| Concentration | Signal | Theoretical signal | Error | Error squared |
|---|---|---|---|---|
| 1.00E-10 | 0.23 | 0.30 | 0.07 | 0.00 |
| 5.24E-11 | 0.25 | 0.36 | 0.11 | 0.01 |
| 2.75E-11 | 0.28 | 0.47 | 0.19 | 0.04 |
| 1.44E-11 | 0.38 | 0.67 | 0.29 | 0.09 |
| 7.56E-12 | 0.54 | 0.97 | 0.43 | 0.19 |
| 3.96E-12 | 0.93 | 1.30 | 0.37 | 0.14 |
| 2.08E-12 | 1.26 | 1.56 | 0.31 | 0.10 |
| 1.09E-12 | 1.66 | 1.74 | 0.08 | 0.01 |
| 5.72E-13 | 1.87 | 1.84 | -0.03 | 0.00 |
| 3.00E-13 | 1.86 | 1.90 | 0.04 | 0.00 |
| 1.57E-13 | 1.96 | 1.93 | -0.03 | 0.00 |
| 8.24E-14 | 1.80 | 1.94 | 0.15 | 0.02 |
| 0.00E+00 | 1.64 | 1.96 | 0.32 | 0.10 |

**FIG 2.1**

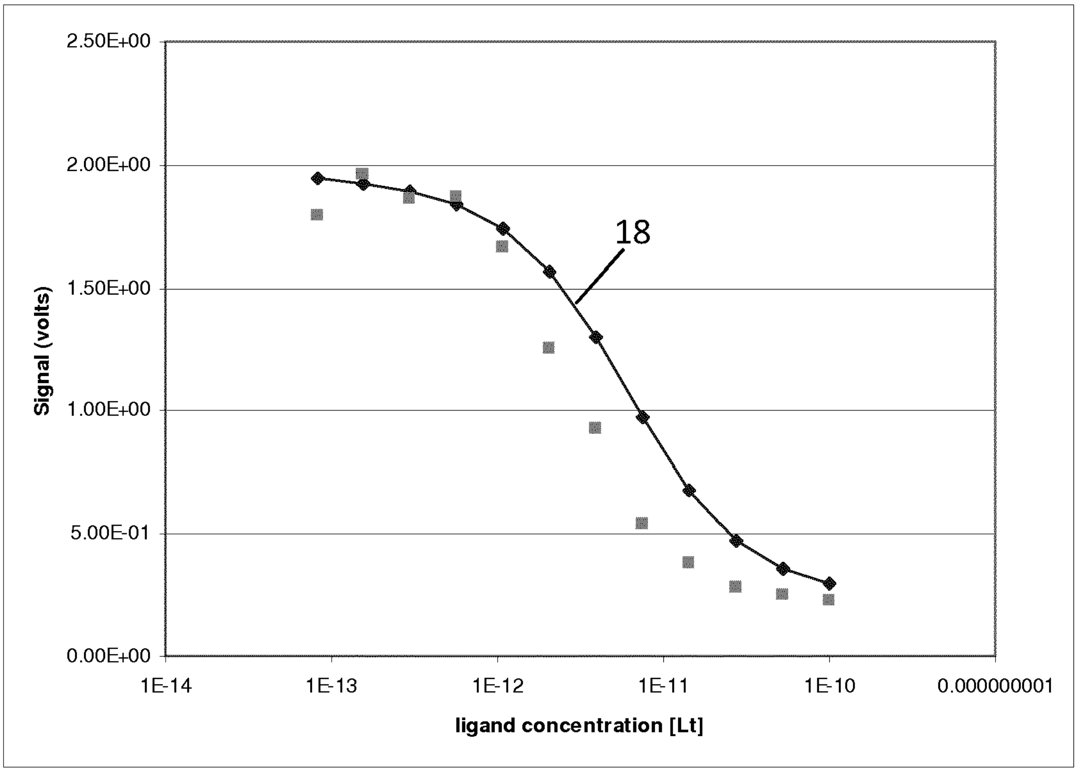


**FIG. 2.2**

| Concentration | Signal | Theoretical signal | Error | Error squared |
|---|---|---|---|---|
| 1.00E-10 | 0.23 | 0.24 | 0.02 | 0.00 |
| 5.24E-11 | 0.25 | 0.26 | 0.01 | 0.00 |
| 2.75E-11 | 0.28 | 0.28 | 0.00 | 0.00 |
| 1.44E-11 | 0.38 | 0.35 | -0.03 | 0.00 |
| 7.56E-12 | 0.54 | 0.53 | 0.00 | 0.00 |
| 3.96E-12 | 0.93 | 0.95 | 0.02 | 0.00 |
| 2.08E-12 | 1.26 | 1.34 | 0.09 | 0.01 |
| 1.09E-12 | 1.66 | 1.59 | -0.07 | 0.00 |
| 5.72E-13 | 1.87 | 1.73 | -0.14 | 0.02 |
| 3.00E-13 | 1.86 | 1.80 | -0.06 | 0.00 |
| 1.57E-13 | 1.96 | 1.84 | -0.12 | 0.01 |
| 8.24E-14 | 1.80 | 1.86 | 0.06 | 0.00 |
| 0.00E+00 | 1.64 | 1.88 | 0.23 | 0.05 |

FIG. 3.1

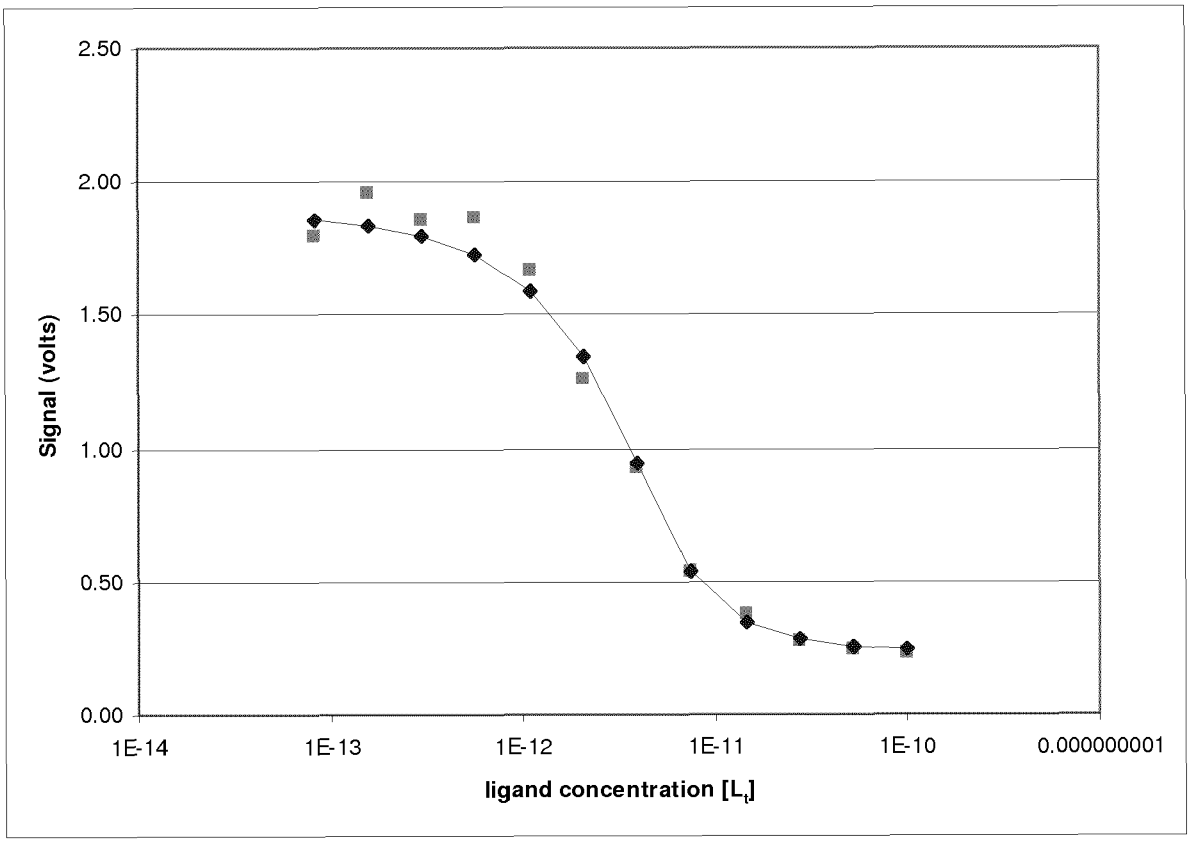


FIG 3.2

| | |
|---|---|
| $K_d$ | 7.16E-13 |
| Rt | 5.36E-12 |
| Sig100 | 1.88 |
| NSB | 0.23 |
| Ratio | 7.48 |
| %Error | 5.67 |

**FIG 3.3**

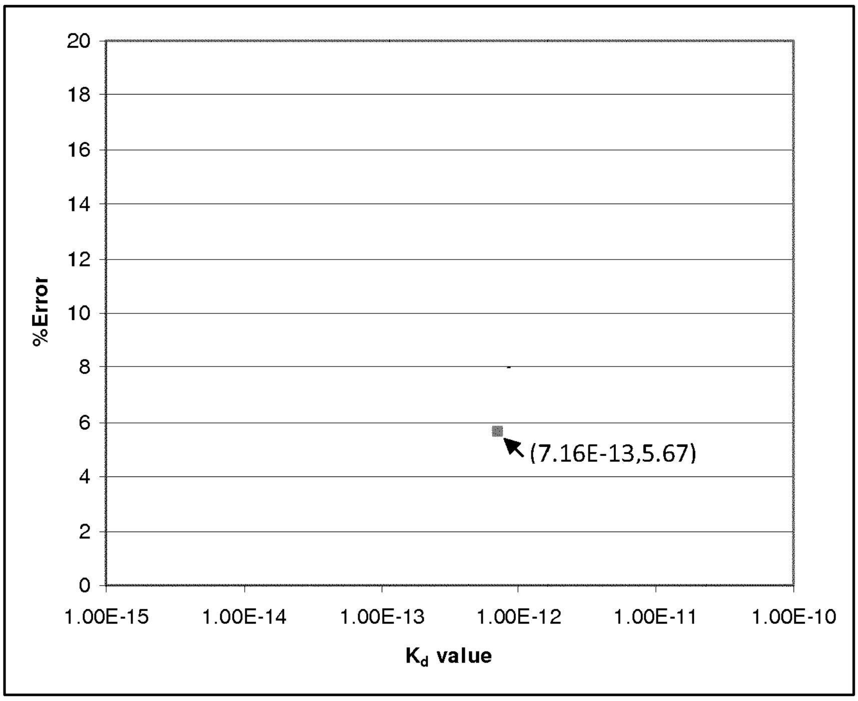


**FIG 4.1**

| | |
|---|---|
| $K_d$ | 7.16E-13 |
| Rt | 5.36E-12 |
| Sig100 | 1.88 |
| NSB | 0.23 |
| Ratio | 7.48 |
| %Error | 5.67 |

**FIG 4.2**

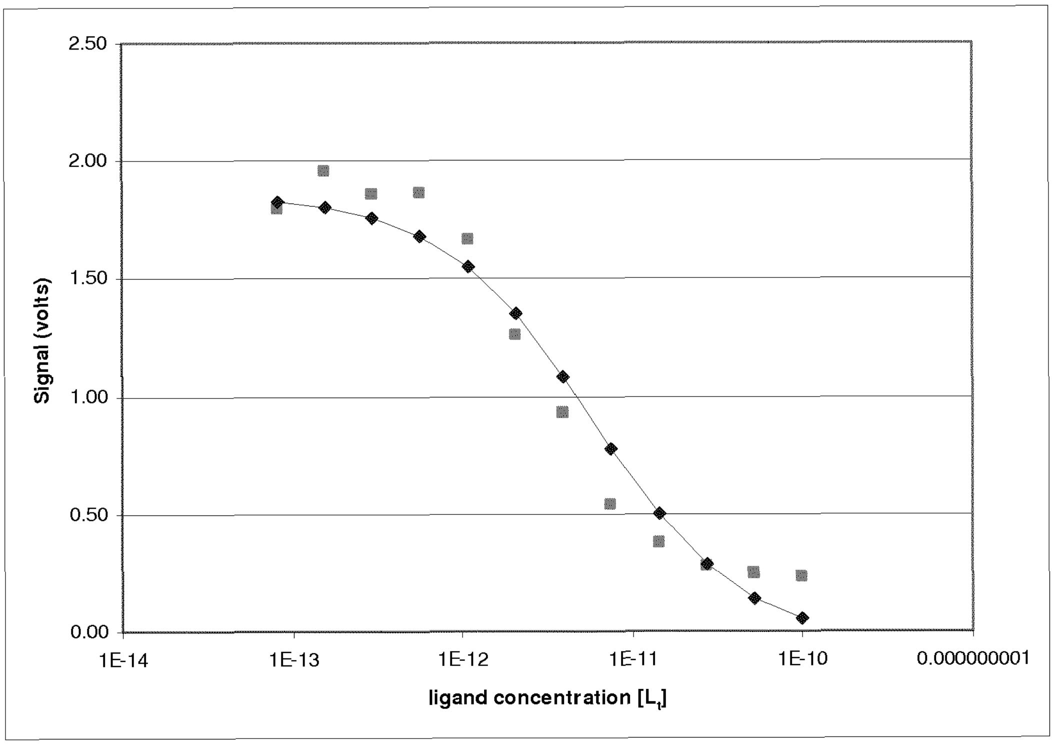
2.50
2.00
1.50
1.00
0.50
0.00
Signal (volts)
1E-14
1E-13
1E-12
1E-11
1E-10
0.000000001
ligand concentration [$L_t$]

FIG. 5.1

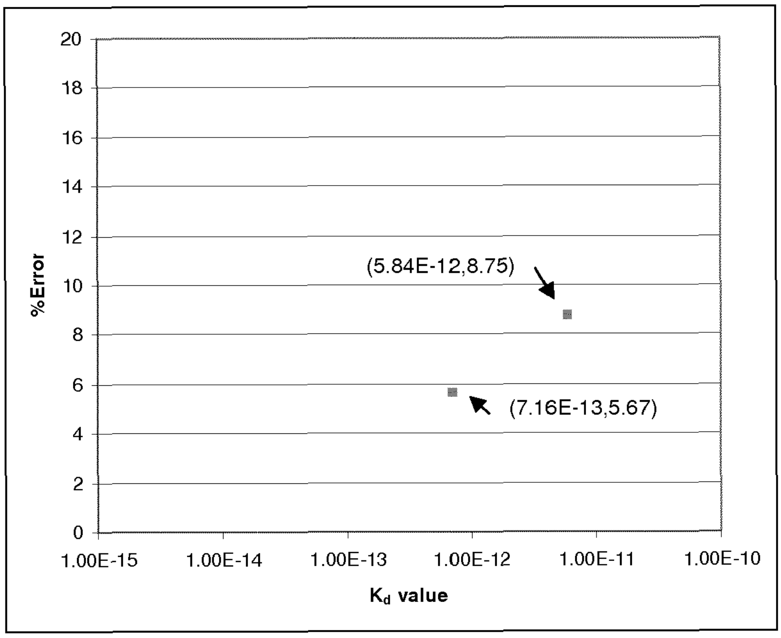
20
18
16
14
12
10
8
6
4
2
0
%Error
(5.84E-12,8.75)
(7.16E-13,5.67)
1.00E-15
1.00E-14
1.00E-13
1.00E-12
1.00E-11
1.00E-10
$K_d$ value

FIG 5.2

| | |
|---|---|
| $K_d$ | 5.84E-12 |
| Rt | 5.59E-15 |
| Sig100 | 1.85 |
| NSB | -0.05 |
| Ratio | 9.57E-04 |
| %Error | 8.75 |

FIG. 5.3

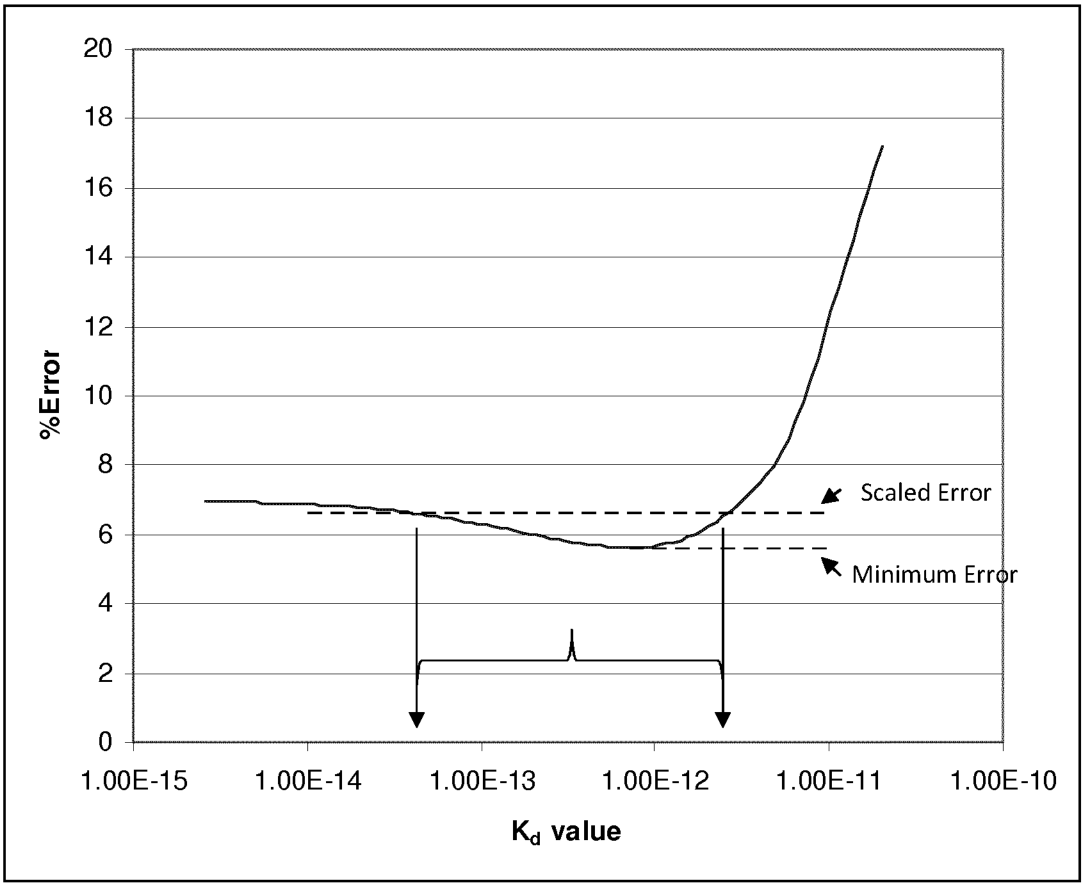


FIG. 6

Kd = 81.35pM
Rt = 48.90pM
Ratio = 0.6011
Sig 100% = 1.89
NSB = 0.25
%Error = 2.82

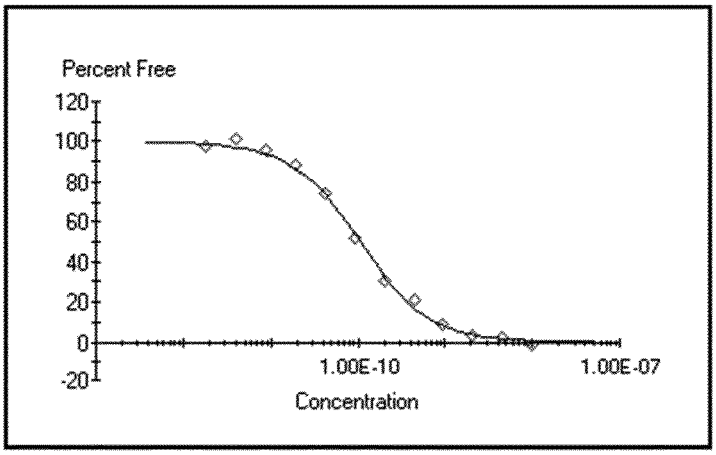


Kd = 81.35pM
95% confidence interval
Kd High = 122.07pM
Kd Low = 49.06pM

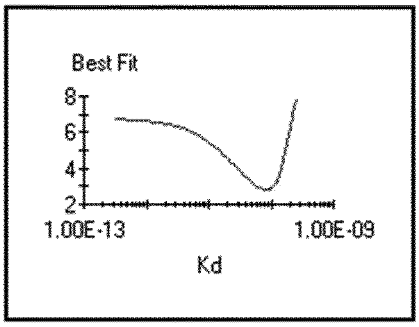


Rt = 48.90pM
95% confidence interval
Rt High = 127.52pM
Rt Low = Less than 176.66fM

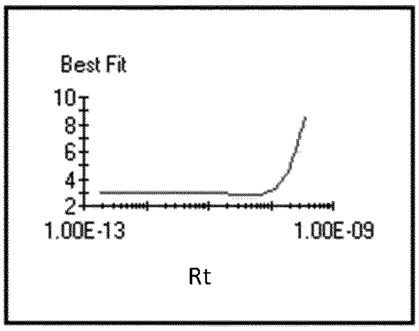


FIG. 7

Kd = 32.13pM
Rt = 10.59nM
Ratio = 329.4880
Sig 100% = 1.93
NSB = 0.22
%Error = 3.52

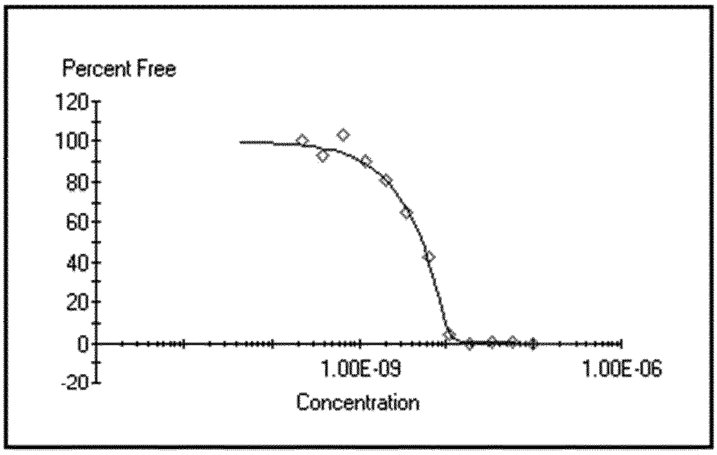


Kd = 32.13pM
95% confidence interval
Kd High = 298.66pM
Kd Low = Less than 116.08fM

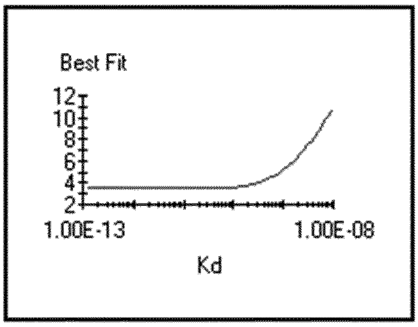


Rt = 10.59nM
95% confidence interval
Rt High = 12.08nM
Rt Low = 8.88nM

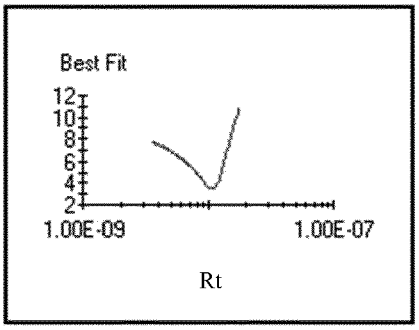


**FIG. 8**

Overall:
Kd = 104.32pM
%Error = 3.27

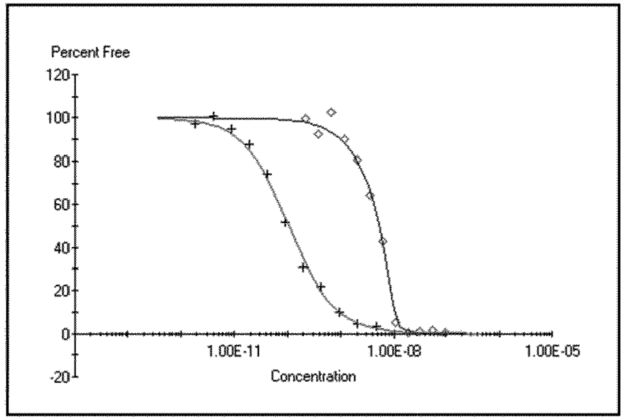


| KinExA Pro Experiment ab controlled sim (Curva 1) | | |
|---|---|---|
| Given Rt | = | 10nM |
| Rt | = | 10.28nM |
| Sig 100% | = | 1.93 |
| NSB | = | 0.21 |
| Ratio | = | 98.544 |

| KinExA Pro Experiment kd controlled sim (Curve 2) | | |
|---|---|---|
| Given Rt | = | 5pM |
| Rt | = | 5.14pM |
| Sig 100% | = | 1.91 |
| NSB | = | 0.23 |
| Ratio | = | 0.049 |

Kd = 104.32pM
95% confidence interval
Kd High = 140.15pM
Kd Low = 77.71pM

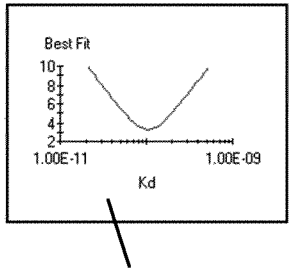


36

Rt = 10.28nM
95% confidence interval
Rt High = 12.20nM
Rt Low = 8.56nM

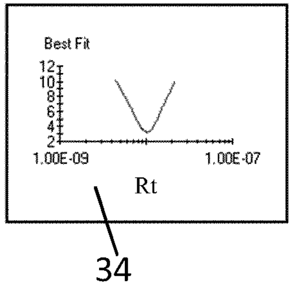


34

FIG. 9

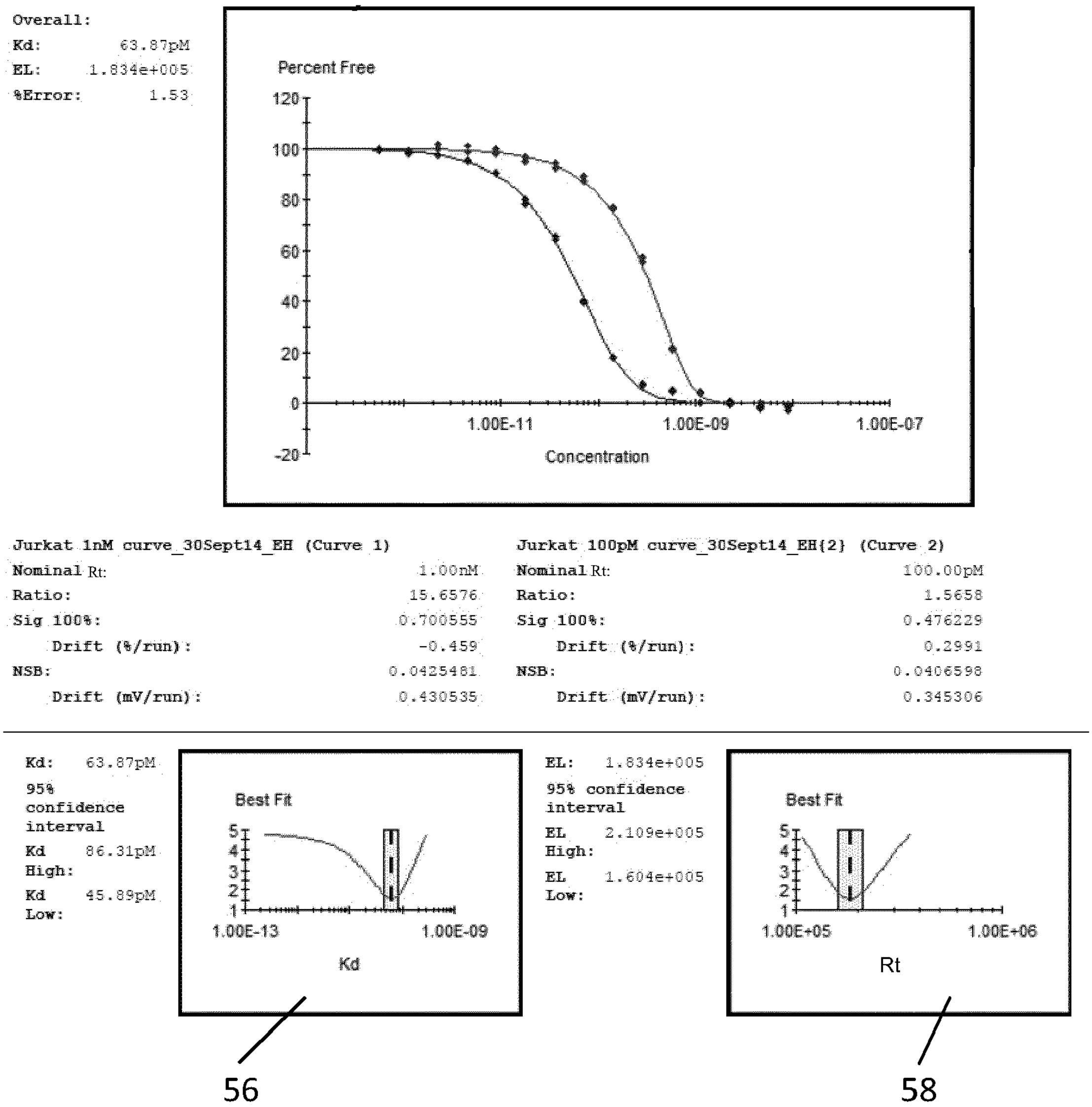
Overall:
Kd: 63.87pM
EL: 1.834e+005
%Error: 1.53
Percent Free
120
100
80
60
40
20
0
-20
1.00E-11
1.00E-09
1.00E-07
Concentration
Jurkat 1nM curve_30Sept14_EH (Curve 1)
Nominal Rt: 1.00nM
Ratio: 15.6576
Sig 100%: 0.700555
Drift (%/run): -0.459
NSB: 0.0425481
Drift (mV/run): 0.430535
Jurkat 100pM curve_30Sept14_EH(2) (Curve 2)
Nominal Rt: 100.00pM
Ratio: 1.5658
Sig 100%: 0.476229
Drift (%/run): 0.2991
NSB: 0.0406598
Drift (mV/run): 0.345306
Kd: 63.87pM
95% confidence interval
Kd High: 86.31pM
Kd Low: 45.89pM
Best Fit
5
4
3
2
1
1.00E-13
1.00E-09
Kd
EL: 1.834e+005
95% confidence interval
EL High: 2.109e+005
EL Low: 1.604e+005
Best Fit
5
4
3
2
1
1.00E+05
1.00E+06
Rt
56
58

FIG. 10

Baseline / Endpoints:
5 to 10 (sec) from beginning
10 to 5 (sec) from end

| | Ignore | Binding Signal (V) | Cells/mL | |
|---|---|---|---|---|
| | | 0.6247 | 0 | |
| 67 | | 0.0353 | 30.00M | 66 |
| | | 0.0383 | 15.00M | |
| | | 0.0428 | 7.50M | |
| | | 0.0678 | 3.75M | |
| | | 0.1730 | 1.88M | |
| | | 0.3885 | 937.50k | |
| | | 0.5216 | 468.75k | |
| | | 0.5962 | 234.38k | |
| | | 0.6295 | 117.19k | |
| | | 0.6593 | 58.59k | |
| | | 0.6839 | 29.30k | 64 |
| | | 0.6947 | 14.65k | |
| | | 0.7032 | 7.32k | |
| | | 0.6902 | 3.66k | |
| | | 0.6910 | 1.83k | |
| | | 0.6923 | 0 | |
| 65 | | 0.0371 | 30.00M | 68 |
| | | 0.0371 | 15.00M | |
| | | 0.0442 | 7.50M | |
| | | 0.0727 | 3.75M | |
| | | 0.1865 | 1.88M | |
| | | 0.4319 | 937.50k | |
| | | 0.5704 | 468.75k | |
| | | 0.6582 | 234.38k | |
| | | 0.6938 | 117.19k | |
| | | 0.7011 | 58.59k | |
| | | 0.7249 | 29.30k | |
| | | 0.7350 | 14.65k | 70 |
| | | 0.7462 | 7.32k | |
| | | 0.7437 | 3.66k | |
| | | 0.7469 | 1.83k | |

**FIG. 11.1**

**Baseline / Endpoints:**
5 to 10 (sec) from beginning
10 to 5 (sec) from end

| Ignore | Binding Signal (V) | Cells/mL |
|---|---|---|
| | 0.0278 | 30.00M |
| | 0.0296 | 15.00M |
| | 0.0396 | 7.50M |
| | 0.0366 | 3.75M |
| | 0.0590 | 1.88M |
| | 0.0698 | 937.50k |
| | 0.1198 | 468.75k |
| | 0.2165 | 234.38k |
| | 0.3241 | 117.19k |
| | 0.3858 | 58.59k |
| | 0.4392 | 29.30k |
| | 0.4602 | 14.65k |
| | 0.4674 | 7.32k |
| | 0.4695 | 3.66k |
| | 0.4760 | 1.83k |
| | 0.4796 | 0 |
| | 0.0275 | 30.00M |
| | 0.0313 | 15.00M |
| | 0.0399 | 7.50M |
| | 0.0425 | 3.75M |
| | 0.0627 | 1.88M |
| | 0.0747 | 937.50k |
| | 0.1201 | 468.75k |
| | 0.2123 | 234.38k |
| | 0.3202 | 117.19k |
| | 0.3824 | 58.59k |
| | 0.4254 | 29.30k |
| | 0.4430 | 14.65k |
| | 0.4522 | 7.32k |
| | 0.4533 | 3.66k |
| | 0.4581 | 1.83k |

**FIG 11.2**

**Experiment Name:** n-Curve Analysis
**Analysis Type:** Equilibrium, Whole Cell

**Overall:**
**Kd:** 149.26pM
**EL:** 2.213e+005
**%Error:** 0.77

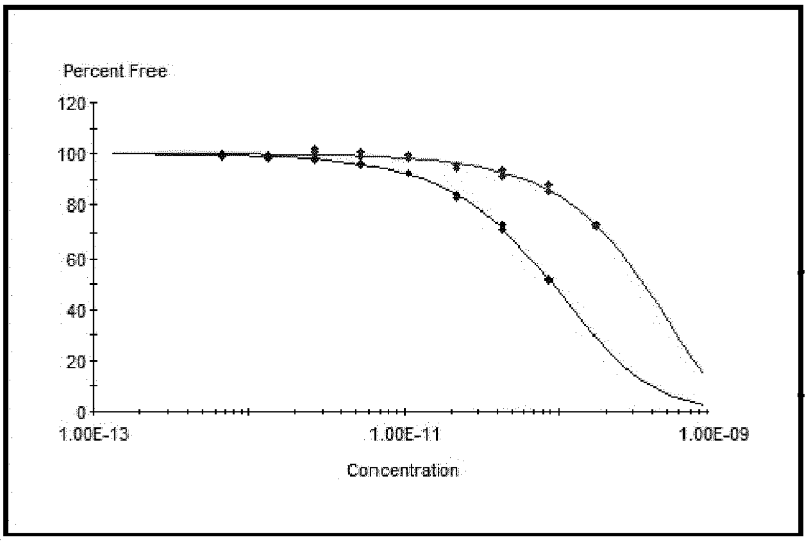


| Jurkat 1nM curve_30Sept14_EH (Curve 1) | | Jurkat 100pM curve_30Sept14_EH(2) (Curve 2) | |
|---|---|---|---|
| Nominal Rt : | 1.00nM | Nominal Rt : | 100.00pM |
| Ratio: | 6.6999 | Ratio: | 0.6700 |
| Sig 100%: | 0.700374 | Sig 100%: | 0.476065 |
| Drift (%/run): | 0 | Drift (%/run): | 0.1985 |
| NSB: | 0.143187 | NSB: | -0.0662813 |
| Drift (mV/run): | 3.15891 | Drift (mV/run): | 0.184426 |

**Kd:** 149.26pM
**95% confidence interval**
**Kd High:** 1.32nM
**Kd Low:** Less than 539.22fM

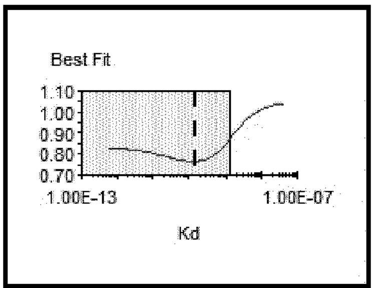


76

**EL:** 2.213e+005
**95% confidence interval**
**EL High:** 1.931e+006
**EL Low:** 6.496e+004

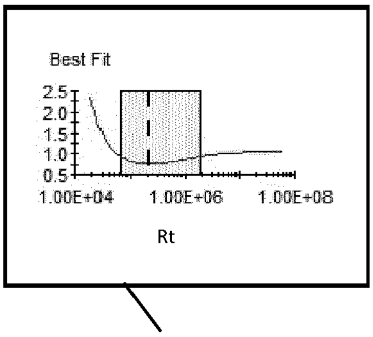


78

**FIG. 12**

**Baseline / Endpoints:**
5 to 10 (sec) from beginning
10 to 5 (sec) from end

**Binding**

| | Ignore | Signal (V) | Cells/mL |
|---|---|---|---|
| | ✓ | 0.6247 | 0 |
| | ✓ | 0.0353 | 30.00M |
| | ✓ | 0.0383 | 100.00M |
| 82 | ✓ | 0.0428 | 7.50M |
| | ✓ | 0.0678 | 3.75M |
| | ✓ | 0.1730 | 1.88M |
| | ✓ | 0.3885 | 937.50k |
| | | 0.5216 | 468.75k |
| | | 0.5962 | 234.38k |
| | | 0.6295 | 117.19k |
| | | 0.6593 | 58.59k |
| | | 0.6839 | 29.30k |
| | | 0.6947 | 14.65k |
| | | 0.7032 | 7.32k |
| | | 0.6902 | 3.66k |
| | | 0.6910 | 1.83k |
| | | 0.6923 | 0 |
| | ✓ | 0.0371 | 30.00M |
| | ✓ | 0.0371 | 100.00M |
| 84 | ✓ | 0.0442 | 7.50M |
| | ✓ | 0.0727 | 3.75M |
| | ✓ | 0.1865 | 1.88M |
| | ✓ | 0.4319 | 937.50k |
| | | 0.5704 | 468.75k |
| | | 0.6582 | 234.38k |
| | | 0.6938 | 117.19k |
| | | 0.7011 | 58.59k |
| | | 0.7249 | 29.30k |
| | | 0.7350 | 14.65k |
| | | 0.7462 | 7.32k |
| | | 0.7437 | 3.66k |
| | | 0.7469 | 1.83k |

**FIG. 13.1**

Baseline / Endpoints:
5 to 10 (sec) from beginning
10 to 5 (sec) from end

Binding

| | Ignore | Signal (V) | Cells/mL |
|---|---|---|---|
| 82 | ✓ | 0.0278 | 30.00M |
| | ✓ | 0.0296 | 15.00M |
| | ✓ | 0.0396 | 7.50M |
| | ✓ | 0.0366 | 100.00M |
| | ✓ | 0.0590 | 1.88M |
| | ✓ | 0.0698 | 937.50k |
| | ✓ | 0.1198 | 468.75k |
| | | 0.2165 | 234.38k |
| | | 0.3241 | 117.19k |
| | | 0.3858 | 58.59k |
| | | 0.4392 | 29.30k |
| | | 0.4602 | 14.65k |
| | | 0.4674 | 7.32k |
| | | 0.4695 | 3.66k |
| | | 0.4760 | 1.83k |
| | | 0.4796 | 0 |
| 84 | ✓ | 0.0275 | 30.00M |
| | ✓ | 0.0313 | 15.00M |
| | ✓ | 0.0399 | 7.50M |
| | ✓ | 0.0425 | 100.00M |
| | ✓ | 0.0627 | 1.88M |
| | ✓ | 0.0747 | 937.50k |
| | ✓ | 0.1201 | 468.75k |
| | | 0.2123 | 234.38k |
| | | 0.3202 | 117.19k |
| | | 0.3824 | 58.59k |
| | | 0.4254 | 29.30k |
| | | 0.4430 | 14.65k |
| | | 0.4522 | 7.32k |
| | | 0.4533 | 3.66k |
| | | 0.4581 | 1.83k |

FIG 13.2

Experiment Name: n-Curve Analysis
Analysis Type: Equilibrium, Whole Cell

Overall:
Kd: 52.18pM
EL: 1.625e+005
%Error: 0.73

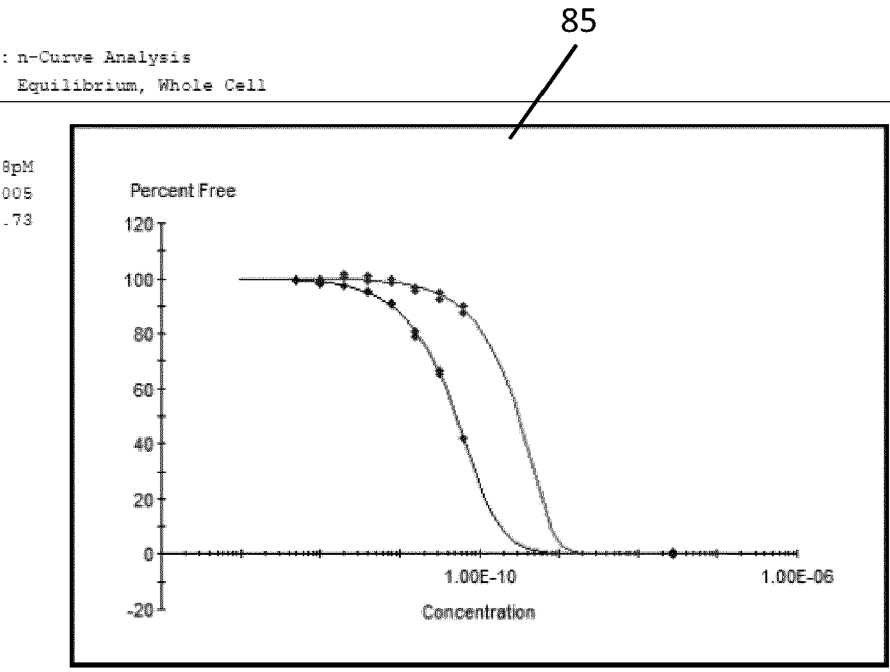


| Jurkat 1nM curve_30Sept14_EH (Curve 1) | | Jurkat 100pM curve_30Sept14_EH(2) (Curve 2) | |
|---|---|---|---|
| Nominal Rt : | 1.00nM | Nominal Rt : | 100.00pM |
| Ratio: | 19.1659 | Ratio: | 1.9166 |
| Sig 100%: | 0.699877 | Sig 100%: | 0.476133 |
| Drift (%/run): | -0.384 | Drift (%/run): | 0.1884 |
| NSB: | 0.0401469 | NSB: | 0.0272017 |
| Drift (mV/run): | 0.625198 | Drift (mV/run): | -0.0648123 |

Kd: 52.18pM
95% confidence interval
Kd High: 84.93pM
Kd Low: 24.24pM

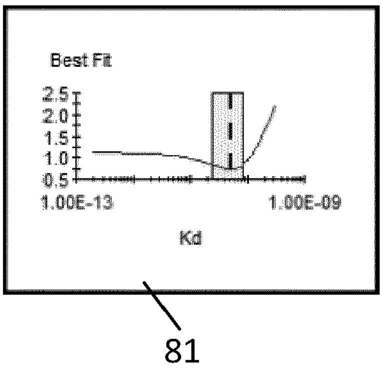


EL: 1.625e+005
95% confidence interval
EL High: 2.137e+005
EL Low: 1.180e+005

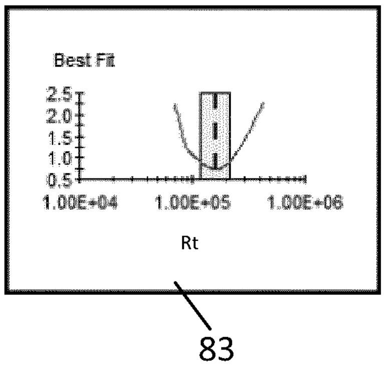


FIG. 14

**Baseline / Endpoints:**
5 to 10 (sec) from beginning
10 to 5 (sec) from end

| Ignore | Binding Signal (V) | Cells/mL | |
|---|---|---|---|
| ✓ | 0.6247 | 0 | |
| | 0.0353 | 100.00M | 96 |
| ✓ | 0.0383 | 15.00M | |
| ✓ | 0.0428 | 7.50M | |
| ✓ | 0.0678 | 3.75M | |
| ✓ | 0.1730 | 1.88M | |
| ✓ | 0.3885 | 937.50k | |
| ✓ | 0.5216 | 468.75k | |
| | 0.5962 | 234.38k | |
| | 0.6295 | 117.19k | |
| | 0.6593 | 58.59k | |
| | 0.6839 | 29.30k | |
| | 0.6947 | 14.65k | |
| | 0.7032 | 7.32k | |
| | 0.6902 | 3.66k | |
| | 0.6910 | 1.83k | |
| | 0.6923 | 0 | |
| | 0.0371 | 100.00M | 97 |
| ✓ | 0.0371 | 15.00M | |
| ✓ | 0.0442 | 7.50M | |
| ✓ | 0.0727 | 3.75M | |
| ✓ | 0.1865 | 1.88M | |
| ✓ | 0.4319 | 937.50k | |
| ✓ | 0.5704 | 468.75k | |
| | 0.6582 | 234.38k | |
| | 0.6938 | 117.19k | |
| | 0.7011 | 58.59k | |
| | 0.7249 | 29.30k | |
| | 0.7350 | 14.65k | |
| | 0.7462 | 7.32k | |
| | 0.7437 | 3.66k | |
| | 0.7469 | 1.83k | |

**FIG. 15.1**

Baseline / Endpoints:
5 to 10 (sec) from beginning
10 to 5 (sec) from end

| Ignore | Binding Signal (V) | Cells/mL | |
|---|---|---|---|
| | 0.0278 | 100.00M | 96 |
| ✓ | 0.0296 | 15.00M | |
| ✓ | 0.0396 | 7.50M | |
| ✓ | 0.0366 | 3.75M | |
| ✓ | 0.0590 | 1.88M | |
| ✓ | 0.0698 | 937.50k | |
| ✓ | 0.1198 | 468.75k | |
| ✓ | 0.2165 | 234.38k | |
| | 0.3241 | 117.19k | |
| | 0.3858 | 58.59k | |
| | 0.4392 | 29.30k | |
| | 0.4602 | 14.65k | |
| | 0.4674 | 7.32k | |
| | 0.4695 | 3.66k | |
| | 0.4760 | 1.83k | |
| | 0.4796 | 0 | |
| | 0.0275 | 100.00M | 97 |
| ✓ | 0.0313 | 15.00M | |
| ✓ | 0.0399 | 7.50M | |
| ✓ | 0.0425 | 3.75M | |
| ✓ | 0.0627 | 1.88M | |
| ✓ | 0.0747 | 937.50k | |
| ✓ | 0.1201 | 468.75k | |
| | 0.2123 | 234.38k | |
| | 0.3202 | 117.19k | |
| | 0.3824 | 58.59k | |
| | 0.4254 | 29.30k | |
| | 0.4430 | 14.65k | |
| | 0.4522 | 7.32k | |
| | 0.4533 | 3.66k | |
| | 0.4581 | 1.83k | |

FIG. 15.2

86

**Experiment Name:** n-Curve Analysis
**Analysis Type:** Equilibrium, Whole Cell

**Overall:**
**Kd:** 47.56pM
**EL:** 1.650e+005
**%Error:** 0.75

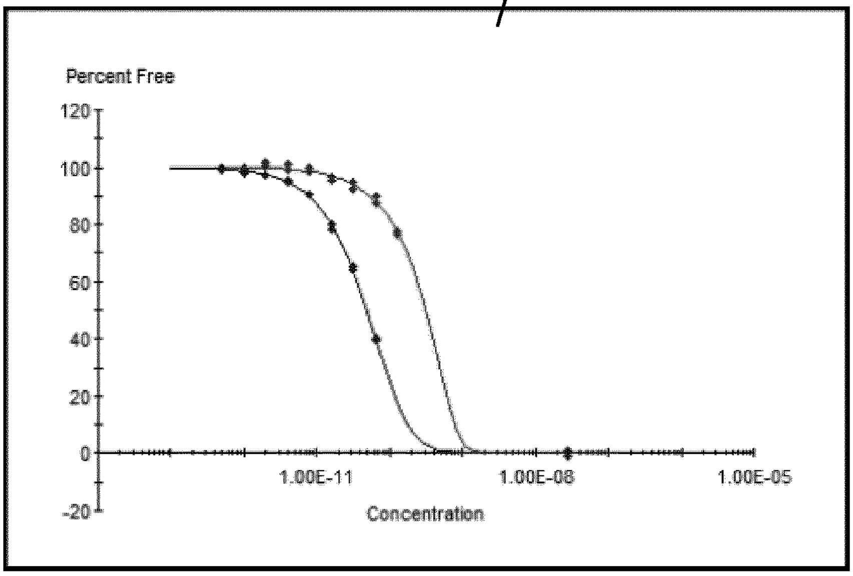


| **Jurkat 1nM curve_30Sept14_EH (Curve 1)** | | **Jurkat 100pM curve_30Sept14_EH(2) (Curve 2)** | |
|---|---|---|---|
| **Nominal** Rt: | 1.00nM | **Nominal** Rt: | 100.00pM |
| **Ratio:** | 21.0247 | **Ratio:** | 2.1025 |
| **Sig 100%:** | 0.699874 | **Sig 100%:** | 0.476802 |
| **Drift (%/run):** | -0.403 | **Drift (%/run):** | 0.3106 |
| **NSB:** | 0.0404664 | **NSB:** | 0.0409188 |
| **Drift (mV/run):** | 0.548181 | **Drift (mV/run):** | 0.364294 |

**Kd:** 47.56pM
**95% confidence interval**
**Kd High:** 65.10pM
**Kd Low:** 32.13pM

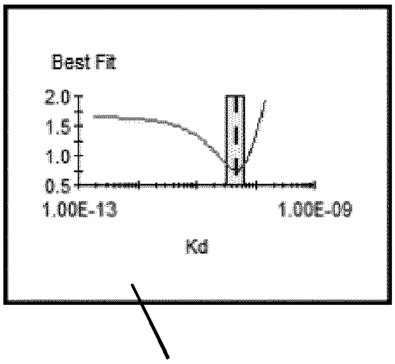


**EL:** 1.650e+005
**95% confidence interval**
**EL High:** 1.923e+005
**EL Low:** 1.398e+005

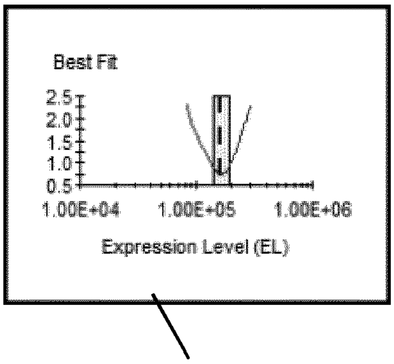


82

84

**FIG. 16**

**Baseline / Endpoints:**
5 to 10 (sec) from beginning
10 to 5 (sec) from end

| Ignore | Binding Signal (V) | Cells/mL | |
|---|---|---|---|
| ✓ | 0.6247 | 0 | |
| ✓ | 0.0353 | 30.00M | |
| | 0.0383 | 100.00M | |
| ✓ | 0.0428 | 7.50M | |
| ✓ | 0.0678 | 3.75M | |
| ✓ | 0.1730 | 1.88M | |
| ✓ | 0.3885 | 937.50k | |
| | 0.5216 | 468.75k | 99 |
| | 0.5962 | 234.38k | |
| | 0.6295 | 117.19k | |
| | 0.6593 | 58.59k | |
| | 0.6839 | 29.30k | |
| | 0.6947 | 14.65k | |
| | 0.7032 | 7.32k | |
| | 0.6902 | 3.66k | |
| | 0.6910 | 1.83k | |
| | 0.6923 | 0 | |
| ✓ | 0.0371 | 30.00M | |
| | 0.0371 | 100.00M | |
| ✓ | 0.0442 | 7.50M | |
| ✓ | 0.0727 | 3.75M | |
| ✓ | 0.1865 | 1.88M | |
| ✓ | 0.4319 | 937.50k | |
| | 0.5704 | 468.75k | 101 |
| | 0.6582 | 234.38k | |
| | 0.6938 | 117.19k | |
| | 0.7011 | 58.59k | |
| | 0.7249 | 29.30k | |
| | 0.7350 | 14.65k | |
| | 0.7462 | 7.32k | |
| | 0.7437 | 3.66k | |
| | 0.7469 | 1.83k | |

**FIG. 17.1**

**Baseline / Endpoints:**
5 to 10 (sec) from beginning
10 to 5 (sec) from end

| | Binding | | |
|---|---|---|---|
| **Ignore** | **Signal (V)** | **Cells/mL** | |
| ✓ | 0.0278 | 30.00M | |
| ✓ | 0.0296 | 15.00M | |
| ✓ | 0.0396 | 7.50M | |
| | 0.0366 | 100.00M | |
| ✓ | 0.0590 | 1.88M | |
| ✓ | 0.0698 | 937.50k | |
| ✓ | 0.1198 | 468.75k | |
| | 0.2165 | 234.38k | 101 |
| | 0.3241 | 117.19k | |
| | 0.3858 | 58.59k | |
| | 0.4392 | 29.30k | |
| | 0.4602 | 14.65k | |
| | 0.4674 | 7.32k | |
| | 0.4695 | 3.66k | |
| | 0.4760 | 1.83k | |
| | 0.4796 | 0 | |
| ✓ | 0.0275 | 30.00M | |
| ✓ | 0.0313 | 15.00M | |
| ✓ | 0.0399 | 7.50M | |
| | 0.0425 | 100.00M | |
| ✓ | 0.0627 | 1.88M | |
| ✓ | 0.0747 | 937.50k | |
| ✓ | 0.1201 | 468.75k | |
| | 0.2123 | 234.38k | 99 |
| | 0.3202 | 117.19k | |
| | 0.3824 | 58.59k | |
| | 0.4254 | 29.30k | |
| | 0.4430 | 14.65k | |
| | 0.4522 | 7.32k | |
| | 0.4533 | 3.66k | |
| | 0.4581 | 1.83k | |

**FIG. 17.2**

Europäisches Patentamt
European Patent Office
Office européen des brevets

# EUROPEAN SEARCH REPORT

Application Number
EP 17 17 1447

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RYAN J. DARLING ET AL: "Kinetic Exclusion Assay Technology: Characterization of Molecular Interactions", ASSAY AND DRUG DEVELOPMENT TECHNOLOGIES, vol. 2, no. 6, 1 December 2004 (2004-12-01), pages 647-657, XP055396049, US ISSN: 1540-658X, DOI: 10.1089/adt.2004.2.647 * the whole document * ----- | 1-11 | INV. G01N33/557 |
| A,D | XIE L ET AL: "Measurement of the functional affinity constant of a monoclonal antibody for cell surface receptors using kinetic exclusion fluorescence immunoassay", JOURNAL OF IMMUNOLOGICAL METHODS, ELSEVIER SCIENCE PUBLISHERS B.V.,AMSTERDAM, NL, vol. 304, no. 1-2, 1 September 2005 (2005-09-01), pages 1-14, XP027659195, ISSN: 0022-1759 [retrieved on 2005-09-01] * the whole document * ----- | 1-11 | TECHNICAL FIELDS SEARCHED (IPC) G01N |
| A | WO 2011/123029 A1 (GE HEALTHCARE BIO SCIENCES AB [SE]; KARLSSON ROBERT [SE]) 6 October 2011 (2011-10-06) * the whole document * ----- -/-- | 1-11 | |

The present search report has been drawn up for all claims

1

| Place of search | Date of completion of the search | Examiner |
|---|---|---|
| Munich | 4 August 2017 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 17 1447

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GLASS THOMAS R ET AL: "Confirmation of the validity of the current characterization of immunochemical reactions by kinetic exclusion assay", ANALYTICAL BIOCHEMISTRY, vol. 456, 2014, pages 38-42, XP029026979, ISSN: 0003-2697, DOI: 10.1016/J.AB.2014.04.011 * the whole document * ----- | 1-11 | |
| | | | TECHNICAL FIELDS SEARCHED (IPC) |

1 The present search report has been drawn up for all claims

| Place of search | Date of completion of the search | Examiner |
|---|---|---|
| Munich | 4 August 2017 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 17 17 1447

This annex lists the patent family members relating to the patent documents cited in the above-mentioned European search report. The members are as contained in the European Patent Office EDP file on
The European Patent Office is in no way liable for these particulars which are merely given for the purpose of information.

04-08-2017

| Patent document cited in search report | Publication date | Patent family member(s) | Publication date |
|---|---|---|---|
| WO 2011123029 A1 | 06-10-2011 | EP 2553457 A1 | 06-02-2013 |
| | | JP 5830523 B2 | 09-12-2015 |
| | | JP 2013524211 A | 17-06-2013 |
| | | US 2013017624 A1 | 17-01-2013 |
| | | WO 2011123029 A1 | 06-10-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **RATHANASWAMI.** High Affinity Binding Measurements of Antibodies to Cell-Surface-Expressed Antigens. *Anal. Biochem.,* 2008, vol. 1, 52-60
- **XIE.** Measurement of the functional affinity constant of a monoclonal antibody for cell surface receptors using kinetic exclusion flourescence immunoassay. *J. Immunological Methods,* 2005, vol. 304, 1-14